# EUROPEAN PATENT SPECIFICATION

(11) **EP 2 677 318 B1**
(45) Date of publication and mention of the grant of the patent: **05.10.2016**
(21) Application number: 12746687.8
(22) Date of filing: 16.02.2012
(51) Int. Cl.: G01N 33/72, G01N 33/66, G01N 33/68

(54) **LABELING AGENT FOR POST-TRANSLATIONAL MODIFICATION ANALYSIS OF SERINE AND THREONINE**
MARKIERUNGSMITTEL FÜR POSTTRANSLATIONALE MODIFIKATIONSANALYSE VON SERIN UND THREONIN
AGENT DE MARQUAGE POUR ANALYSE DES MODIFICATIONS POST-TRADUCTIONNELLES DE LA SÉRINE ET DE LA THRÉONINE

(30) Priority: 16.02.2011 JP 2011031420
(43) Date of publication of application: 25.12.2013
(73) Proprietor: Cellseed Inc., Tokyo 135-0064 (JP); National University Corporation Hokkaido University, Sapporo-shi, Hokkaido 060-0808 (JP)
(72) Inventor: SHINOHARA, Yasuro, Hokkaido 060-0808 (JP); TAKEGAWA, Yasuhiro, Hokkaido 060-0808 (JP); FUJITANI, Naoki, Hokkaido 060-0808 (JP); FURUKAWA Jun-ichi, Hokkaido 060-0808 (JP); SAKAI, Hideaki, Tokyo 162-0056 (JP)
(74) Representative: Hoffmann Eitle
(86) International application number: PCT/JP2012/053718
(87) International publication number: WO 2012/111775

(56) References cited:
- EP-A1- 0 480 751
- WO-A1-03/078401
- WO-A1-03/080583
- JP-A- 2007 515 625
- FARWELL D C ET AL: "Methods for the identification of N-asparaginyl and O-seryl/threonyl glycosidic linkages to aminosugars in glycoproteins", ANALYTICAL BIOCHEMISTRY, ACADEMIC PRESS INC, NEW YORK, vol. 113, no. 2, 15 May 1981 (1981-05-15), pages 423-437, XP024826436, ISSN: 0003-2697, DOI: 10.1016/0003-2697(81)90097-X [retrieved on 1981-05-15]
- AMINOFF D ET AL: "Quantitation of oligosaccharides released by the beta-elimination reaction", ANALYTICAL BIOCHEMISTRY, ACADEMIC PRESS INC, NEW YORK, vol. 101, no. 1, 1 January 1980 (1980-01-01), pages 44-53, XP024824069, ISSN: 0003-2697, DOI: 10.1016/0003-2697(80)90038-X [retrieved on 1980-01-01]
- WELLS L ET AL: "Mapping sites of )-GlcNac modification using affinity tags for serine and threonine post translational modifications", MOLECULAR & CELLULAR PROTEOMICS, AMERICAN SOCIETY FOR BIOCHEMISTRY AND MOLECULAR BIOLOGY, vol. 1, 1 September 2002 (2002-09-01), pages 791-804, XP003002616, ISSN: 1535-9476, DOI: 10.1074/MCP.M200048-MCP200
- LATTOVA E ET AL: "Influence of the Labeling Group on Ionization and Fragmentation of Carbohydrates in Mass Spectrometry", JOURNAL OF THE AMERICAN SOCIETY FOR MASS SPECTROMETRY, ELSEVIER SCIENCE INC, US, vol. 16, no. 5, 1 May 2005 (2005-05-01), pages 683-696, XP025303024, ISSN: 1044-0305, DOI: 10.1016/J.JASMS.2005.01.021 [retrieved on 2005-05-01]
- JUN-ICHI FURUKAWA ET AL: "A Versatile Method for Analysis of Serine/Threonine Posttranslational Modifications by [beta]-Elimination in the Presence of Pyrazolone Analogues", ANALYTICAL CHEMISTRY, vol. 83, no. 23, 1 December 2011 (2011-12-01), pages 9060-9067, XP055134989, ISSN: 0003-2700, DOI: 10.1021/ac2019848
- SUSUMU HONDA: "Application of capillary electrophoresis to the analyses of carbohydrates and glycoproteins.", SEIBUTSU BUTSURI KAGAKU = JOURNAL OF ELECTROPHORESIS, vol. 40, no. 3, 1 January 1996 (1996-01-01), pages 147-154, XP055125950, JP ISSN: 0031-9082
- SUSUMU HONDA: 'Application of capillary electrophoresis to the analyses of carbohydrates and glycoproteins' JOURNAL OF ELECTROPHORESIS vol. 40, no. 3, 1996, pages 147 - 154, XP055125950
- YOSHINAO WADA ET AL.: 'Tackling Difficulties in the Determination of O-Glycosylation Sites : Approaches to Mucin-type Glycoproteins' TRENDS GLYCOSCIENCE GLYCOTECHNOLOGY vol. 20, no. 112, 2008, pages 69 - 80, XP008170301
- SUSUMU HONDA: 'Carbohydrate analysis and carbohydrate-protein binding studies by capillary/microchip electrophoresis' JOURNAL OF ELECTROPHORESIS vol. 46, no. 2, 2002, pages 63 - 73, XP003030086
- KAKEHI K: 'A Simple Method for Derivatization of Complex Oligosaccharides Derived from Glycoconjugates with 1-(4-Methoxy)Phenyl-3- Methyl-5-Pyrazolone' TRENDS GLYCOSCIENCE GLYCOTECHNOLOGY vol. 4, no. 19, 1992, pages 479 - 482, XP003030087
- ETHIER M ET AL.: 'Automated structural assignment of derivatized complex N-linked oligosaccharides from tandem mass spectra' RAPID COMMUN MASS SPECTROM vol. 16, no. 18, 2002, pages 1743 - 1754, XP002902846
- VOCADLO D J ET AL.: 'A chemical approach for identifying O-GlcNAc-modified proteins in cells' PROC NATL ACAD SCI USA vol. 100, no. 16, 2003, pages 9116 - 9121, XP002987353
- CARLSON DM: 'Structures and Immunochemical Properties of Oligosaccharides Isolated from Pig Submaxillary Mucins' J BIOL CHEM vol. 243, no. 3, 1968, pages 616 - 626, XP055120402
- MANIATIS STEPHANIE ET AL.: 'Rapid De-O- Glycosylation Concomitant with Peptide Labeling Using Microwave Radiation and an Alkyl Amine Base' ANAL CHEM vol. 82, no. 6, 2010, pages 2421 - 2425, XP055120404
- WADA YOSHINAO ET AL.: 'Comparison of methods for profiling 0-glycosylation: Human Proteome Organisation Human Disease Glycomics/Proteome Initiative' MOLECULAR & CELLULAR PROTEOMICS vol. 9, no. 4, 2010, pages 719 - 727, XP055120408

## Description

### TECHNICAL FIELD

The present invention relates to a novel process for analyzing post-translational modification groups attached to serine and threonine, which is useful in various fields including biochemistry, biology, pharmacy, drug discovery, and medicine. In particular, the present invention involves a labeling agent for selectively cleaving O-glycans, from serine and threonine in post-translationally modified glycoproteins and/or glycopeptides and for labeling said groups.

### BACKGROUND ART

It is believed that proteins in the living body exhibit a variety of physiological activities as a result of diverse post-translational modifications, such as phosphorylation, sulfation, or sugar chain modification, made to proteins translated by genes. Among these modifications, phosphorylation and sulfation are common post-translational modifications, which impart physiological activities and functions to many proteins. In particular, glycosylation is a topic that is recently attracting a lot of attention, and the modified sugar chains impart various functions to proteins. For example, some proteins do not fold correctly before they have been glycosylated. There is known an example where oligosaccharides are attached to asparagines, thereby conferring stability to proteins and controlling the metabolic fates of glycoproteins in the body. It is also believed that glycosylation plays a role in intercellular adhesion. Sugar chains that modify proteins are broadly divided into three types: N-glycan, O-glycan, and glycosaminoglycan (GAG). The types of monosaccharides constituting the sugar chains as found in higher organisms are believed to include mannose (Man), glucose (Glc), galactose (Gal), xylose (Xyl), glucuronic acid (GlcA), N-acetylglucosamine (GIcNAc), N-acetylgalactosamine (GalNAc), fucose (Fuc), and sialic acid (SA). The N-glycan has a sugar chain transferred through a N linkage to asparagine (Asn) in a peptide having the sequence -asparagine-X-threonine or - asparagine-X-serine (where X is an amino acid residue other than proline (Pro)). The O-glycan has its first sugar transferred through an O linkage to a threonine (Thr) residue or a serine (Ser) residue. The first sugar of many O-glycans is N-acetylgalactosamine but that of some O-glycans is mannose, fucose, N-acetylglucosamine, or the like. In many cases, following the first sugar, further sugars are transferred one after another, so that an O-glycan sugar chain is elongated. Proteoglycan takes a form in which a large number of very long sugar chains are added to a protein. The sugar chain moiety thereof having amino sugars (N-acetylglucosamine or N-acetylgalactosamine) is referred to as glycosaminoglycan (GAG). The glycosaminoglycan is characterized by its very long sugar chains having a repeating structure of disaccharide units.

Here, serine and threonine are amino acid residues that undergo diverse post-translational modifications such as phosphorylation, sulfation, and sugar chain modification (e.g., O-GalNAcylation, O-GlcNAcylation, O-fucosylation, O-manosylation, O-xylosylation, O-galactosylation, O-glucosylation), and the post-translational modifications of these residues play an important role in controlling various high-order functions of life, such as signaling and differentiation control. For example, kinase inhibitors that are responsible for phosphorylation of particular proteins have been already placed on the market as pharmaceutical products, and qualitative and quantitative changes in O-linked sugar chains are applied to the diagnosis of various diseases. Among the post-translational modifications of serine and threonine, O-phosphorylation, O-sulfation, or O-GlcNAcylation refers to the attachment of a phosphate group, a sulfate group, or an O-GlcNAc group, respectively, alone to an amino acid residue, whereas it is generally known that O-linked sugar chain modifications such as O-GalNAcylation, O-fucosylation, O-manosylation, and O-xylosylation provide further elongated sugar chains with diverse structures.

In this connection, the β-elimination and Michael addition (BEMA) method is available as one of processes for cleaving and labeling the phosphorylation-binding sites of phosphorylated serine and phosphorylated threonine. This method is established by applying the fact that under a basic condition, a phosphate group is β-eliminated to produce unsaturated carbonyl, which in turn serves as a Michael reaction acceptor (refer to Patent Documents 1 and 2, and Non-patent Document 1). For example, the phosphorylation site can be labeled by causing a thiol group-containing compound such as dithiothreitol to act thereon, and this technique is utilized for various purposes including peptide purification and on-bead/on-chip fluorescence labeling (refer to Patent Document 3). The BEMA method contributes to and is widely used in studies about protein phosphorylation, but in this method, other post-translational modification groups including an O-GlcNAcylation group which are attached to serine and threonine are also cleaved, so it is impossible to distinguished which modification groups were present in what kind of state before cleavage. Further, in the BEMA method, a thiol group-containing compound is commonly used as a Michael reaction acceptor and there are few cases where any other Michael donors are applied, which indicates that this method has a limited application range. There is another report about a process for analyzing proteins that have undergone O-GlcNAcylation, by which metabolic labeling is performed with a non-native GlcNAc derivative having an azido group at the 2-position (Non-patent Document 2). Furthermore, in the case of analysis of diverse structures of an O-GalNAcylated (mucin-type) sugar chain moiety, β-elimination under strong alkali conditions is widely used for cleavage of the sugar chains, because no effective enzyme has been discovered for cleaving the sugar chains from a protein. In this method, the released sugar chains can also undergo β-elimination sequentially from the side of their reducing terminals, giving rise to degradation (peeling reaction) and thus a high-concentration reducing agent is added to ensure that the reducing terminals of the sugar chains are reduced to sugar alcohol upon β-elimination. This method was reported by Carlson in 1968 and is still used as a standard process for releasing O-GalNAcylated sugar chains (Non-patent Document 3). However, this method has such problems as follows: (1) it is difficult to analyze a protein moiety because β-elimination in the presence of a reducing agent gives rise to degradation of a peptide moiety, and (2) it is difficult to perform derivatization for sugar chain separation or highly sensitive analysis because reduction causes loss of the reducing terminals of sugar chains. Accordingly, there has been a strong demand for the development of a methodology of releasing O-linked sugar chains from peptides and proteins in high yields without degrading the sugar chains and of performing derivatization for separation and highly sensitive analysis.

WO 03/078401 A1 relates to preventive and/or therapeutic agents for hypoxic ischemic brain disorder.

WO 03/080583 A1 relates to a medicine for the prevention and/or therapy of cardiomyopathy. EP 0 480 751 A1 relates to a method for labelling sugars.

Analytical biochemistry, 113( 2), 423-437 (1981) relates to methods for the identification of N-asparaginyl and O-seryl/threonyl glycosidic linkages to aminosugars in glycoproteins. Analytical biochemistry, 101(1), 44-53 (1980) relates to the quantitation of oligosaccharides released by the beta-elimination reaction.

Molecular & cellular proteomics, 1, 791-804 (2002) relates to mapping sites of )-GlcNac modification using affinity tags for serine and threonine post translational modifications. Journal of the American society for mass spectrometry, 16(5) 683-696 (2005) relates to the influence of the labeling group on ionization and fragmentation of carbohydrates in mass spectrometry.

Analytical chemistry, 83(23), 9060-9067 (2011) relates to a versatile method for analysis of serine/threonine posttranslational modifications by [beta]-elimination in the presence of pyrazolone analogues.

Journal of electrophoresis, 40(3), 147-154 (1996) relates to the application of capillary electrophoresis to the analyses of carbohydrates and glycoproteins.

### CITATION LIST

### PATENT DOCUMENT

[Patent Document 1] Japanese Patent Domestic Publication No. 2007-515625
[Patent Document 2] U.S. Patent No. 7803751
[Patent Document 3] Japanese Unexamined Patent Application Publication No. 2009-19002
[Patent Document 4] U.S. Patent Publication No. 2006-0099604 A1
[Patent Document 5] U.S. Patent Publication No. 2005-01.76093 A1
[Patent Document 6] U.S. Patent Publication No. 2005-0095725 A1
[Patent Document 7] U.S. Patent Publication No. 2005-0014197 A1
[Patent Document 8] U.S. Patent Publication No. 2004-0038306 A1
[Patent Document 9] U.S. Patent Publication No. 2003-0219838 A1

### NON-PATENT DOCUMENT

[Non-patent Document 1] Nature Biotechnology, 19 (4), 379-382 (2001)
[Non-patent Document 2] Proceedings of the National Academy of Sciences of the United States of America, 100 (16), 9116-9121 (2003)
[Non-patent Document 3] J, Biol. Chem., 243, 616-626 (1968)

### SUMMARY OF THE INVENTION

### TECHNICAL PROBLEM

Protein structure analysis techniques are progressing and there are increasing opportunities to analyze various post-translational modification groups, whereas in the conventional art, only the O-phosphate groups post-translationally attached to serine and threonine have been analyzed by the BEMA method mentioned above. In the case of analysis of other post-translational modifications by O-sulfate groups, O-glycans, and the like, this BEMA method enabled cleavage of these modification groups but did not allow subsequent labeling of said groups, so it was impossible to analyze, for example, the position of the amino acid residue to which an O-glycan has been attached or the structure of the O-glycan. An object of the present invention is to solve these problems and, more specifically, to provide a process for selectively cleaving and effectively analyzing post-translational modification O-glycans using labeling agent that is capable of universally labeling these post-translational modification groups attached to serine residues and threonine residues in post-translationally modified glycoproteins and/or glycopeptides. Other objects of the invention are to provide a process for obtaining the information on the types of modifications and the modification positions on peptides, to provide a process for chemically modifying post-translational modification sites and simultaneously recovering released sugar chains as any given derivatives that are advantageous for separation and detection without causing a peeling reaction of said sugar chains, and to provide a new compound group for labeling a peptide moiety by the BEMA method.

### SOLUTION TO PROBLEM

In order to solve the above-mentioned problems, the present inventors have made research and development with considerations being given from various perspectives. As a result, the inventors have found that when under a basic condition, a particular labeling agent is added to an O-glycan post-translationally attached to serine or threonine and heat is applied, the post-translational modification group can be selectively cleaved and the cleaved post-translational modification group can be effectively labeled. This invention has been completed based on these findings. This invention provides an analysis process that is superior to conventional processes in terms of simplicity and selectivity, and which makes it possible to selectively cleave post-translational modification groups from serine residues and/or threonine residues in various post-translationally modified glycoproteins and/or glycopeptides and to analyze said modification groups. This invention further enables analysis of the glycoproteins and/or glycopeptides from which said modification groups are cleaved.

The present disclosure relates to a labeling agent for selectively cleaving a post-translational modification group from a serine residue and/or a threonine residue in a post-translationally modified glycoprotein and/or glycopeptide under a basic condition, and for labeling at least one member of the serine residue, the threonine residue, and the post-translational modification group selectively cleaved from said amino acid residues. This invention also provides a process for cleaving a post-translational modification group, wherein a glycoprotein and/or a glycopeptide which are a test substance as well as the labeling agent are dissolved or dispersed in a solvent and heat is applied under a basic condition, thereby selectively cleaving the post-translational modification group from a serine residue and/or threonine residue in the test substance, wherein the labelling agent comprises, as active ingredient, at least a pyrazolone derivative and wherein the post-translational modification group is an O-glycan. This invention further provides a process for analyzing the post-translational modification group attached to the serine residue and/or the threonine residue of the glycoprotein and/or the glycopeptide, using said post-translational modification group cleavage process. This invention is believed to be a very important invention based on a novel idea unique in the world, which enables comprehensive analysis of O-glycans post-translationally attached to serine and threonine -- such an analysis has been impossible with conventional techniques.

### ADVANTAGEOUS EFFECTS OF INVENTION

### The process of the present invention makes it possible not only to release

O-glycans from glycoproteins with simple operations, under mild conditions, and without the use of a hazardous reagent, but also to label both the released sugar chains and the remaining peptides with the same reagent or different reagents. This labeling contributes to various factors including easiness of subsequent separation and/or improved sensitivity in detection, thereby enabling easy purification of the labeled sugar chains and peptides. This also enables highly sensitive structural analysis and quantitative analysis of the labeled sugar chains and peptides by liquid chromatography or mass spectrometry and, thus, allows comprehensive analysis of post-translational modifications of serine and threonine.

### BRIEF DESCRIPTIONS OF THE DRAWINGS

[FIG. 1] FIG. 1 shows the MALDI-TOF spectrum results obtained when samples of the glycopeptide model having O-GalNAcylated sugar chains were heated in the presence of PMP, DP, and MTP, respectively, under a basic condition in Example 1.
[FIG. 2] FIG. 2 shows the reaction path presumed in Example 1.
[FIG. 3] FIG. 3 shows the profiles of the O-linkedsugar chains cleaved from the glycopeptide model having O-GalNAcylated sugar chains under the following different conditions in Example 2: A) reductive β-elimination (Carlson method); B) β-Elimination Kit (Sigma), 4°C, 24 hours; C) β-Elimination Kit (Sigma), room temperature, 24 hours; D) dimethylamine, 50°C, 16 hours; E) dimethylamine, 70°C, 16 hours; and F) PMP under a basic condition, 70°C, 16 hours.
[FIG. 4] FIG. 4 shows the TOF/TOF spectrum taken in Example 3 from the peptides labeled with DP in Example 1.
[FIG. 5] FIG. 5 shows the TOF/TOF spectrum taken in Example 3 from the peptides labeled with PMP in Example 1.
[FIG. 6] FIG. 6 shows the TOF/TOF spectrum taken in Example 3 from the peptides labeled with MTP in Example 1.
[FIG. 7] FIG. 7 shows the MALDI-TOF spectra of the products generated when samples of the glycopeptide model having O-GalNAcylated sugar chains were heated in the presence of DTT, DP, and DTT/DP, respectively, under a basic condition in Example 4.
[FIG. 8] FIG. 8 shows the reaction path presumed for the case where a sample of the glycopeptide model having O-GalNAcylated sugar chains was heated in the presence of DTT/DP under a basic condition in Example 4.
[FIG. 9] FIG. 9 shows the profiles of the O-linkedsugar chains obtained when bovine fetuin and porcine gastric mucin were each heated in the presence of DP under a basic condition in Example 5. In this figure, a circle (o) represents galactose, a light-colored square (□) represents N-acetylgalactosamine, a deep-colored square (□) represents N-acetylglucosamine, a triangle (Δ) represents fucose, and a diamond (◊) represents N-acetylneuraminic acid.
[FIG. 10] FIG. 10 shows the MALDI-TOF spectra of the products generated when the phosphorylated peptide model (left panels) and the O-GlcNAcylated peptide model (right panels) were each heated in the presence of PMP or DP under a basic condition in Example 6 (the upper panels show the spectra of the starting materials, and the lower panels show those of the products).
[FIG. 11] FIG. 11 shows the MALDI-TOF spectra of the reaction products generated when samples of the glycopeptide model having O-GalNAcylated sugar chains were heated in the presence of DTT, DP, and DTT+DP, respectively, under a basic condition in Example 7.
[FIG. 12] FIG. 12 shows the mass spectra obtained when a pyrazolone derivative (DP) and/or dithiothreitol (DTT) was/were added to each sample of glycopeptides having O-GalNAcylated sugar chains under a basic condition in Example 7, and the respective mixtures were heated at 70°C for 16 hours.
[FIG. 13] FIG. 13 shows the profiles of the O-linkedsugar chains obtained when bovine fetuin and porcine submandibular gland mucin were each heated in the presence of DP under a basic condition. In this figure, a circle (o) represents galactose, a light-colored square (□) represents N-acetylgalactosamine, a deep-colored square (□) represents N-acetylglucosamine, a triangle (Δ) represents fucose, and a diamond (◊) represents N-acetylneuraminic acid.
[FIG. 14] FIG. 14 shows the spectra of the sugar chains labeled with novel pyrazolone reagents (the upper, middle, and lower panels show the spectra of the sugar chains labeled with Compound 5, 10, and 14, respectively).
[FIG. 15] FIG.15 shows the reaction scheme representing the synthesis path of an arginine-added pyrazolone reagent.
[FIG.16] FIG. 16 shows the reaction scheme representing the synthesis path of another arginine-added pyrazolone reagent.
[FIG.17] FIG. 17 shows the reaction scheme representing the synthesis path of a biotin-added pyrazolone reagent.

### DESCRIPTION OF EMBODIMENT

The present invention is directed to a technique for analyzing post-translational modifications involved by serine residues and/or threonine residues in post-translationally modified glycoproteins and/or glycopeptides. The origin of the glycoproteins and/or glycopeptides to be used for the analysis is not limited at all, and examples include animals such as humans, rats, mice, guinea pigs, marmosets, rabbits, dogs, cats, sheep, pigs, chimpanzees or those animals with immunodeficiency, or plants, but in cases where the results of the present invention are used for treatment of humans, cells derived from humans, pigs, and chimpanzees are preferably used. As described above, this invention provides a means for analyzing post-translational modification groups attached to amino acids, i.e., serine residues and/or threonine residues having a hydroxy group, in post-translationally modified glycoproteins and/or glycopeptides. These post-translational modification groups are glycans, which are attached to an amino acid through its oxygen atom.

In particular, the glycans each attached to an. amino acid through its oxygen atom (hereinafter, these glycans maybe referred to as "O-glycans" ) can be any sugar that is capable of binding to an oxygen atom and which starts from any of N-acetylgalactosamine, mannose, fucose, N-acetylglucosamine, xylose, and glucose, or can be any product in which a sugar that is capable of binding to an oxygen atom and which starts from any of N-acetylgalactosamine, mannose, fucose, N-acetylglucosamine, xylose, and glucose is conjugated with an additional sugar to be elongated.

In the present invention, it is necessary that such a post-translational modification group attached to a serine residue and/or a threonine residue be cleaved from said serine residue and/or said threonine residue. The reaction to be performed for the cleavage can be any β-elimination reaction according to routine methods, and the conditions for the reaction are not particularly limited; examples include a method by which a post-translationally modified glycoprotein and/or glycopeptide is dissolved or dispersed in a solvent and heat is applied under a basic condition. The β-elimination conditions are exemplified by standing in lithium hydroxide, sodium hydroxide, or barium hydroxide with a concentration of 10 mM to 1 M at about 4-60°C for about 10 minutes to 24 hours.

In the present invention, a double-bond generated upon the release of the post-translational modification group attached to serine or threonine by β-elimination is labeled by Michael addition. The Michael addition is generally performed in one pot by adding a Michael addition donor such as a pyrazolone derivative with a concentration of about 20 mM to 1M upon β-elimination. The addition also enables labeling of the released post-translational modification group. In this invention, the serine residue and/or the threonine residue may be labeled alone, or both the residues and the modification group may be labeled, or the serine residue and/or the threonine residue as well as the modification group may be labeled with different reagents. The labelling agent used in the process of the present invention comprises, as active ingredient, at least a pyrazolone derivative. The reagents to be used in the Michael addition reaction are not particularly limited; examples include thiol compounds, pyrazolone derivatives, isoxazolone derivatives, hydantoin derivatives, rhodanine derivatives, and maleimide derivatives.

In particular, pyrazolone derivatives, isoxazolone derivatives, hydantoin derivatives, rhodanine derivatives, maleimide derivatives, and the like are advantageous in this invention because they can perform labeling simultaneously with the Michael addition reaction. The labeling agent, as referred to in this invention, means a formulation containing such a reagent. Further, in this invention, pyrazolone derivatives are comprised as active ingredient in the labelling agent of this invention. They are not particularly limited as long as they are compounds having a pyrazolone group, and examples include 3-methyl-1-phenyl-5-pyrazolone (PMP), 1,3-dimethyl-pyrazolone (DP), 3-methyl-1-p-tolyl-5-pyrazolone (MTP), and 3-methyl-1-(quinolin-8-yl)-1H-pyrazol-5(4H)-one. Exemplary isoxazolone derivatives include 3(2H)-isoxazolone, 5-methyl-3(2H)-isoxazolone, and 2,5-dimethyl-3(2H)-isoxazolone. Exemplary hydantoin derivatives include 2,4-imidazolidinedione, 3-methyl-2,4-imidazolidinedione, and 3-(2-propyn-1-yl)-2,4-imidazolidinedione. Exemplary rhodanine derivatives include 2-thioxo-4-thiazolidinone and 3-methyl-2-thioxo-4-thiazolidinone.

In the present invention, any of the labeling agents mentioned above may be used in combination with a thiol compound. The thiol compound to be used in the process is not particularly limited as long as it is a compound having a thiol group, and examples include dithiothreitol (DTT), 2-aminoethanethiol, thiocholine, and 2-(pyridin-4-yl)ethanethiol. Depending on the combination of the labeling agents, labeling takes place on the serine residue and/or the threonine residue alone or on both the residues and the post-translational modification group as described above, so the labeling agent(s) to be used can be selected as appropriate depending on the purpose. This is specifically explained using the above-mentioned labeling agents, 1,3-dimethyl-pyrazolone (DP) and dithiothreitol (DTT), as examples. In the case where the post-translational modification group is a sugar chain, it is labeled when DP is added, but it is not labeled when DTT is added. When both DP and DTT are added, only the DP-labeled sugar chain is detected. As for the protein or peptide, the DTT-labeled protein/peptide is detected when DTT is added, and the DP-labeled protein/peptide when DP is added. When DTT and DP are used in combination, only DTT reacts with the protein or peptide and DP does not react with it. These facts are known from experience.

In the present invention, the state of the reaction mixture to be produced upon cleavage of a post-translational modification group from a glycoproteins and/or a glycopeptide which are a test substance is not particularly limited, and they may be either dissolved or dispersed in a solvent. In this invention, it is advantageous to perform a reaction under the condition where the glycoprotein and/or the glycopeptide as well as the labeling agent are dissolved in water, because the reaction can be run effectively. Further, the reaction mixture may be stirred to more effectively run the reaction.

In the present invention, once a post-translational modification group is cleaved from a serine residue and/or a threonine residue in a test substance and the post-translational modification group and/or the test substance is/are labeled by the process described above, said post-translational modification group and/or said test substance can be analyzed by liquid chromatography or high performance liquid chromatography. Other appropriate assays can also be applied depending on the type of the molecule of interest, and examples of those assays which can be used include mass spectrometry (MS) and nuclear magnetic resonance (NMR) spectroscopy, as well as analyses with an ultraviolet (UV) absorptiometer, an evaporative light scattering (ELS) detector, and an electrochemical detector. In this invention, any one of these assays may be used alone, or two or more of them may be used in combination.

The present invention enables highly sensitive structural analysis and quantitative analysis of post-translational modifications of test substances, i.e., glycoproteins and/or glycopeptides, as well as comprehensive analysis of post-translational modifications of serine and threonine.

### EXAMPLES

The present invention will be described below in more detail by way of Examples, but these Examples do not limit the invention at all.

### [Example 1]

### (Test Description 1)

Glycopeptides having O-GalNAcylated sugar chains were synthesized as previously reported. To each aqueous solution of the glycopeptides (4 µg/mL, 5 µL), 45 µL of water, 50 µL of 0.6 M NaOH, as well as 100 µL of a methanol solution of 0.5 M 3-methyl-1-phenyl-5-pyrazoione (PMP), or an aqueous solution of 0.5 M 1,3-dimethyl-5-pyrazolone (DP), or a methanol solution of 0.5 M 3-methyl-1-p-tolyl-5-pyrazolone (MTP)was added respectively, and the respective mixtures were allowed to stand at 70°C for 16 hours. After completion of the reaction, the reaction mixtures were neutralized with 0.3 M hydrochloric acid and directly mixed with 2,5-dihydrobenzoic acid (10 mg/mL), and the mixtures were analyzed by MALDI-TOF(/TOF) (UltraFlex II, Bruker).

### (Test Description 2)

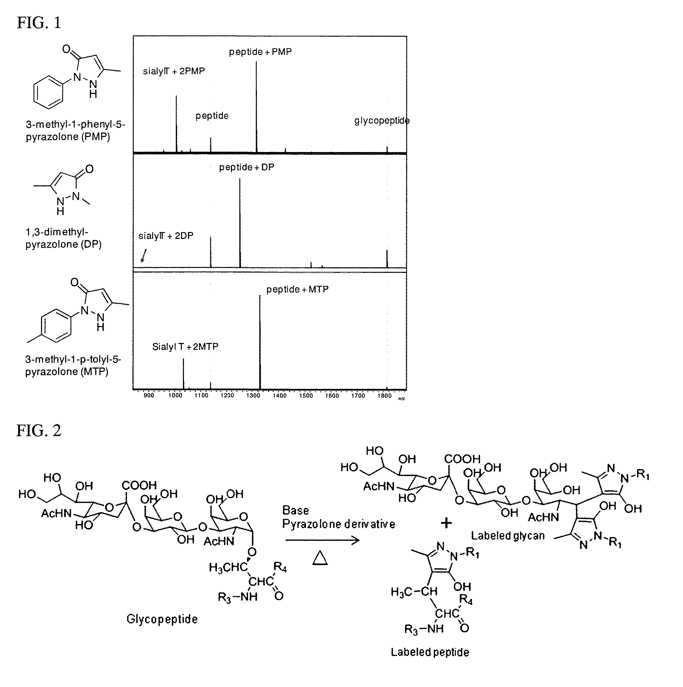

Next, three different types of pyrazolone derivatives were added individually to different samples of the glycopeptides having O-GalNAcylated sugar chains under a basic condition, and the respective mixtures were heated at 70°C for 16 hours to degrade the glycopeptides. More specifically, 10 µL of 1 M NaBH₄ (100 mM NaOH solution) and 5 µL of water were added to an aqueous solution of the glycopeptides having O-GalNAcylated sugar chains (4 µg/mL, 5 µL), and the mixture was allowed to stand at 50°C for 15 hours (Sample A). 12 µL of water and 3.4 µL of β-Elimination Reagent Mix (Sigma) were added to other aqueous solutions of the glycopeptides (4 µg/mL, 5 µL), and the mixtures were allowed to stand at 4°C or room temperature for 24 hours (Samples B, C). 18 µL of dimethylamine was added to still other aqueous solutions of the glycopeptides (4 µg/mL, 5 µL), and the mixtures were allowed to stand at 50°C or 70°C for 16 hours (Samples D, E). 45 µL of water, 50 µL of 0.6 M NaOH, and 100 µL of a methanol solution of 0.5 M 3-methyl-1-phenyl-5-pyrazolone (PMP) were added to yet another aqueous solution of the glycopeptides (4 µg/mL, 5 µL), and the mixture was allowed to stand at 70°C for 16 hours (Sample F). After completion of the reaction, the respective reaction mixtures were neutralized, provided however that as for Sample A, a 1% acetate/methanol solution was added and the mixture was repeatedly evaporated to dryness. Samples A-E were subjected to purification of their sugar chain moieties using Dowex 50X8 and ZipTipC18, and analyzed by MALDI-TOF(/TOF) (UltraFlex II, Bruker) using 2,5-dihydrobenzoic acid (10 mg/mL) as a matrix. Sample F was analyzed for its sugar chains by the procedure described in Test Description 1. The obtained mass spectrometry results are shown in FIG. 1. In every sample, the signal of the starting material, i.e., glycopeptides, significantly decreased in intensity and the signals of the peptides and sugar chains labeled with a pyrazolone derivative appeared instead. The presumed reaction is shown in FIG. 2.

### [Example 2]

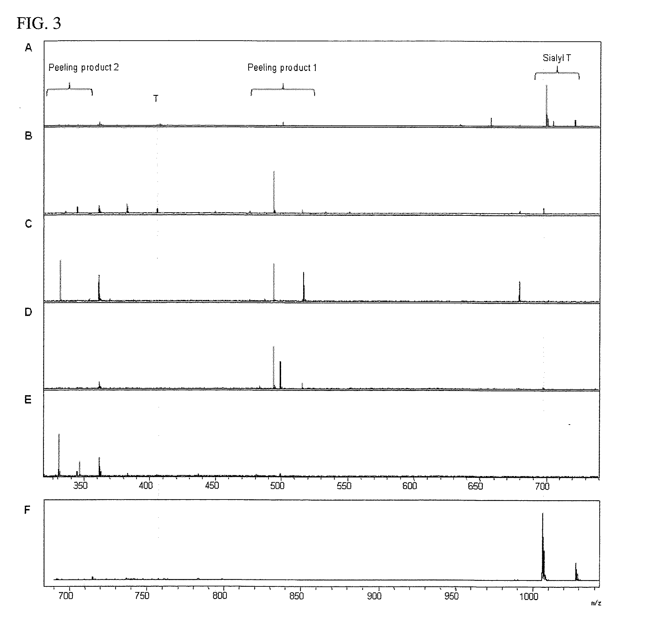

Glycopeptides having O-GalNAcylated sugar chains were subjected to release of the sugar chains by various existing methods, and the sugar chain profiles obtained during these processes were compared. The obtained mass spectrometry results are shown in FIG. 3. The glycopeptides used were found to mainly comprise trisaccharides called "siaryl-T" (Neu5Acα2-3Galβ1-3GalNAc) and contain trace amounts of disaccharides called "T-antigen" (Galβ1-3GalNAc). FIG. 3A shows the sugar chain profile obtained by the Carlson method in which simultaneously with p-elimination, the reducing terminals of the sugar chains were reduced to sugar alcohol in the presence of a reducing agent (NaBH₄) to prevent degradation of the sugar chains. The peaks for siaryl-T and T-antigen were observed but those for the degradation products were present only in trace amounts. FIGs. 3B and 3C show the results obtained by releasing the sugar chains under the two recommended types of conditions using the β-elimination kit commercially available from Sigma. The sugar chain release method using dimethylamine (Anal. Chem., 2010, 82, 2421-2425) was also performed under two types of conditions (FIGs. 3D and 3E). In any of these instances, the degradation products from a peeling reaction accounted for a very large percentage; sugar chain release without reduction resulted in significant deterioration in the sugar chain profiles. The tendency for the sugar chain profiles to deteriorate upon sugar chain release without reduction was also noted in a recent paper by the Human Proteome Organization (Mol. Cel. Proteomics, 2010, 9, 719-727). On the other hand, when PMP was added under a basic condition, the only peaks detected were for the siaryl-T and trace T-antigen which were labeled with PMP, and no peaks were detected at all for any degradation products (FIG. 3F).

These results demonstrated that even in the case of releasing O-linkedsugar chains by β-elimination under a basic condition, a pyrazolone derivative, if any, can quickly react with the sugar chains to give stable labeled products while giving little degradation products.

### [Example 3]

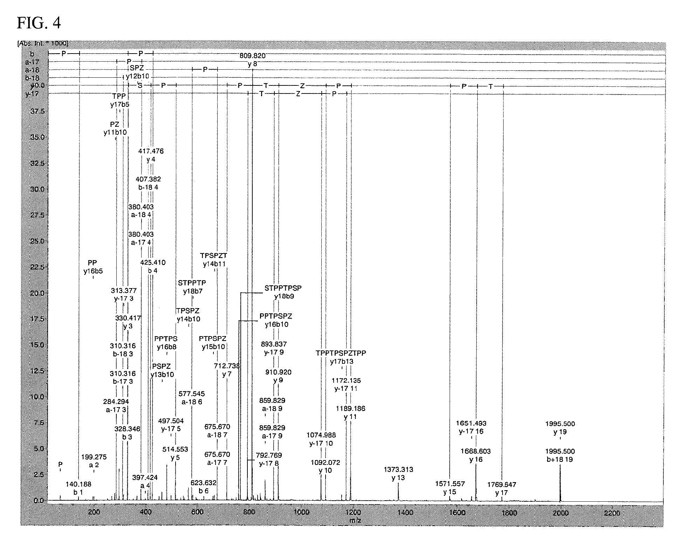

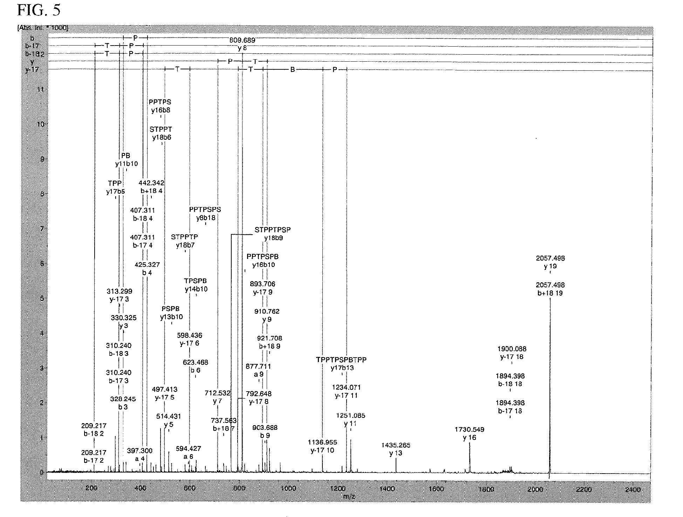

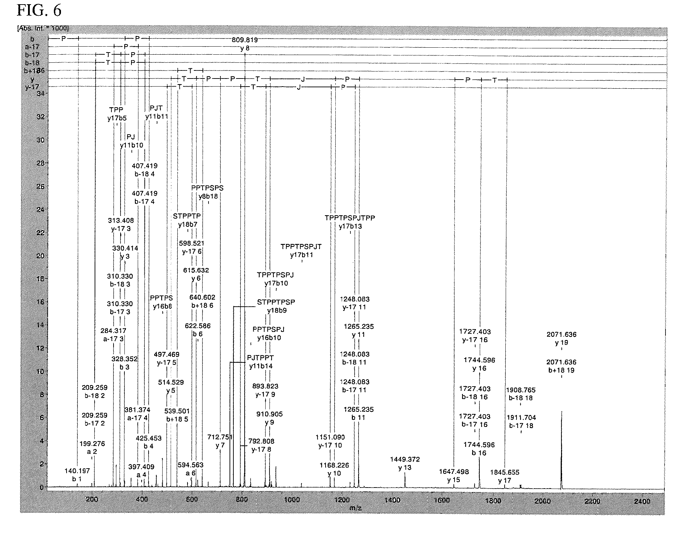

FIGs. 4, 5 and 6 respectively show the TOF/TOF spectra of the peptides labeled with DP, PMP, or MTP, respectively in Example 1. Any of these samples gave satisfactory information on peptide sequences and about the modification positions of the sugar chains. These results demonstrated that β-elimination reaction in the presence of a pyrazolone derivative satisfactorily yields the information on the structures and sequences of the sugar chains and peptides.

### [Example 4]

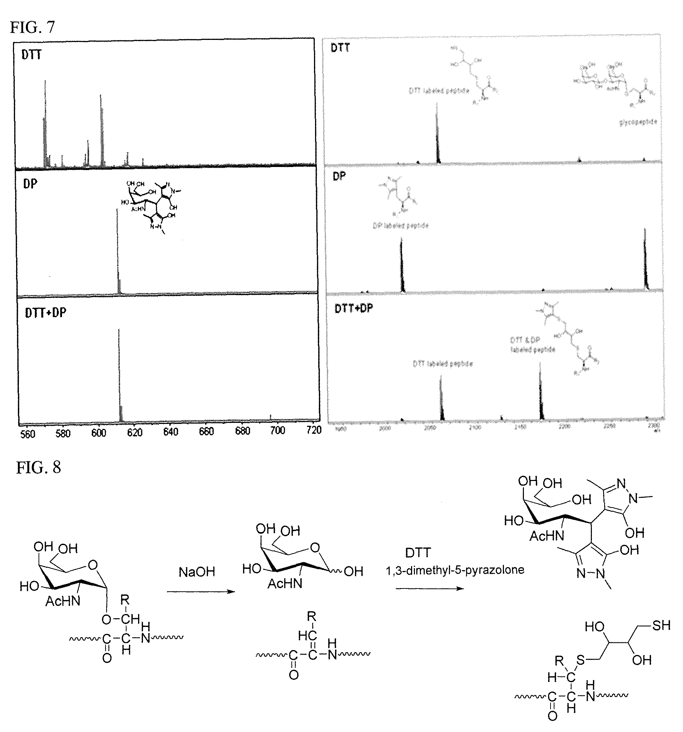

A pyrazolone derivative (DP) and/or dithiothreitol (DTT) was/were added to each sample of the glycopeptides having O-GalNAcylated sugar chains under a basic condition, and the respective mixtures were heated at 70°C for 16 hours. More specifically, to each sample of the glycopeptides having O-GalNAcylated sugar chains (4 µg/mL, 5 µL), 5 µL of 0.6 M NaOH was first added and then 10 µL of 0.5 M DTT, or 10 µL of 0.5 M DP, or 5 µL of 0.5 M DTT plus 5 µL of 0.5 M DP was/were added; thereafter, the respective mixtures were heated at 70°C for 16 hours. After completion of the reaction, the respective reaction mixtures were diluted and mixed with DHB, and the mixtures were analyzed by MALDI-TOF(/TOF) (UltraFlex II, Bruker). The obtained mass spectrometry results are shown in FIG. 7. The sugar chains were labeled only when DP was added (upper left panel in FIG. 7), but they were not labeled when DTT was added (middle left panel in FIG. 7). When both DP and DTT were added, only the DP-labeled sugar chains were detected (lower left panel in FIG. 7; FIG. 8). As for the peptides, when DTT was added, the DTT-labeled peptides were detected, and when DP was added, the DP-labeled peptides were detected (upper and middle right panels in FIG. 7). When both DTT and DP were added, only the peptides that reacted with DTT were detected, with no products of direct reaction with DP being detected (lower right panel in FIG. 7, FIG. 8). These results demonstrated that sugar chains and peptides can be each selectively labeled with different reagents.

### [Example 5]

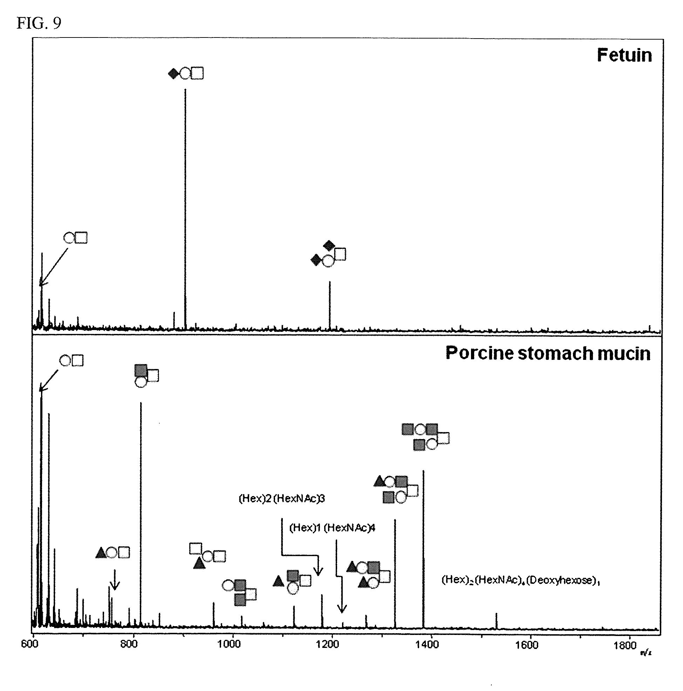

Bovine fetuin, porcine gastric mucin, and human IgA, which were known to have O-linked sugar chains, were profiled for their O-linkedsugar chains using the technique as mentioned in the present invention. The results are shown in FIG. 9. 50 µg each of bovine fetuin, porcine gastric mucin, or human IgA was weighed out, and 50 µL of water, 100 µL of a 0.5 M DP solution, and 50 µL of 0.6 M NaOH were added to each of the samples; thereafter, the respective mixtures were allowed to stand at 70°C for 16 hours. After completion of the reaction, the respective reaction mixtures were neutralized, and a quarter of each reaction mixture (50 µL) was subjected to purification of its sugar chain moiety using Dowex 50X8 and ZipTipC18. After evaporated to dryness, the resulting products were each dissolved in DHB and then analyzed by MALDI-TOF(/TOF) (UltraFlex II, Bruker). Any of the results were qualitatively and quantitatively comparable to those previously reported. This demonstrated that the present invention is useful for analysis of O-linkedsugar chains in glycoproteins. As for the bovine fetuin, which was known to contain both O-linkedsugar chains and N-linked sugar chains, only the O-linkedsugar chains were detected according to the inventive technique, with no N-linked sugar chains detected at all. These results demonstrated that the technique of this invention is not effective on N-linked sugar chains but has a highly selective effect on O-linkedsugar chains.

### [Example 6]

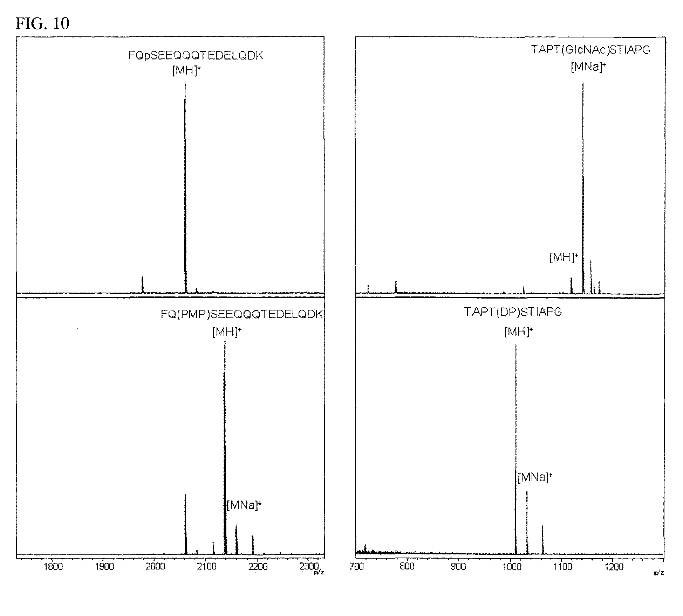

The technique of the present invention was applied to two different types of peptides having phosphorylation modification or O-GlcNAcylated sugar chain modification and mass spectra showing the results were taken (FIG. 10). More specifically, phosphorylated glycopeptides (FQpSEEQQQTEDELQDK) derived from bovine β-casein were purchased from AnaSpec. O-GlcNAcylated peptides (TAPT (O-GlcNAc) STIAPG) were purchased from Invitrogen. To 50 µg (5 µL) of the phosphorylated glycopeptides, 10 µL of 0.5 M PMP and 5 µL of 0.6 M NaOH were added, and the mixture was allowed to stand at 70°C for 16 hours. To 5.6 µg (10 µL) of the O-GlcNAcylated peptides, 20 µL of 0.5 M DP and 10 µL of 0.6 M NaOH were added, and the mixture was allowed to stand at 70°C for 16 hours. The resulting reaction mixtures were each directly mixed with DHB and then analyzed by MALDI-TOF. As a result, both of the starting materials, i.e., modified peptides, significantly decreased after the reaction -- the peptides were converted in high yields by the respective pyrazolone derivatives added. These results demonstrated that the inventive technique has the same effect on phosphorylation of serine and threonine, and O-GlcNAcylated sugar chain modification, as on O-GalNAcylated sugar chain modification.

### [Example 7]

To each aqueous solution of glycopeptides having O-GalNAcylated sugar chains (4 µg/mL, 5 µL), 5 µL of 0.6 M NaOH was first added and then 10 µL of 0.5 M DTT, or 10 µL of 0.5 M DP, or 5 µL of 0.5 M DTT plus 5 µL of 0.5 M DP was/were added; thereafter, the respective mixtures were heated at 70°C for 16 hours. After completion of the reaction, the respective reaction mixtures were diluted and mixed with DHB, and the mixtures were analyzed by MALDI-TOF(/TOF) (UltraFlex II, Bruker).

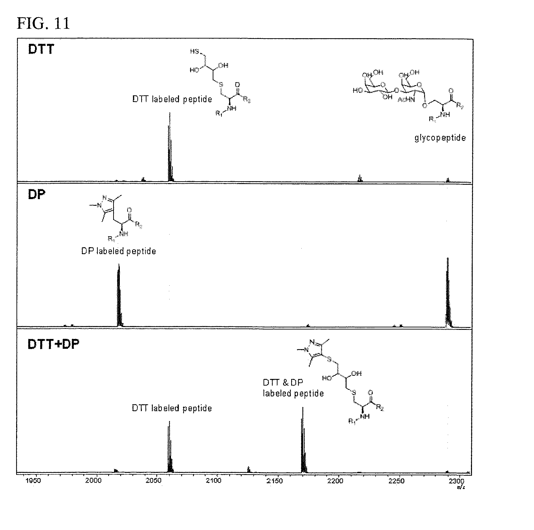

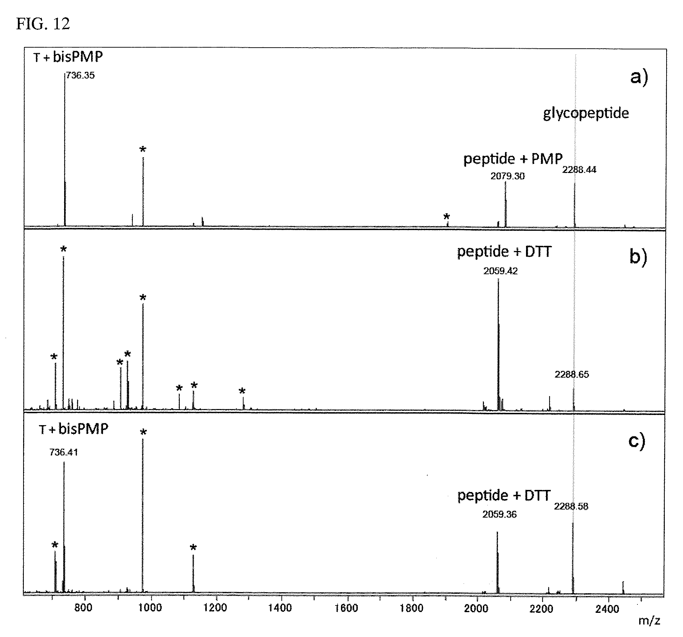

To each aqueous solution of glycopeptides having O-GalNAcylated sugar chains (4 µg/mL, 10 µL), 20 µL of 0.45 M NaOH was first added and then 20µL of 0.5 M PMP, or 20 µL of 0.5 M DTT, or 10 µL of 1.0 M DTT plus 10 µL of 1.0 M DPPMP was/were added; thereafter, the respective mixtures were heated at 85°C for 16 hours. After completion of the reaction, the respective reaction mixtures were diluted and mixed with DHB, and the mixtures were analyzed by MALDI-TOF(/TOF) (UltraFlex II, Bruker). The results are shown in FIGs. 11 and 12.

FIG. 12 shows the mass spectra obtained when a pyrazolone derivative (DP) and/or dithiothreitol (DTT) was/were added to each sample of glycopeptides having O-GalNAcylated sugar chains under a basic condition, and the respective mixtures were heated at 70°C for 16 hours. The sugar chains were labeled only when DP was added, but they were not labeled when DTT was added. When both DP and DTT were added, only the DP-labeled sugar chains were detected. As for the peptides, when DTT was added, the DTT-labeled peptides were detected, and when DP was added, the DP-labeled peptides were detected (upper and middle panels in FIG. 11). When both DTT and DP were added, only the peptides that reacted with DTT were detected, with no products of direct reaction with DP being detected (lower panel in FIG. 11). These results demonstrated that sugar chains and peptides can be each selectively labeled with different reagents.

### [Example 8]

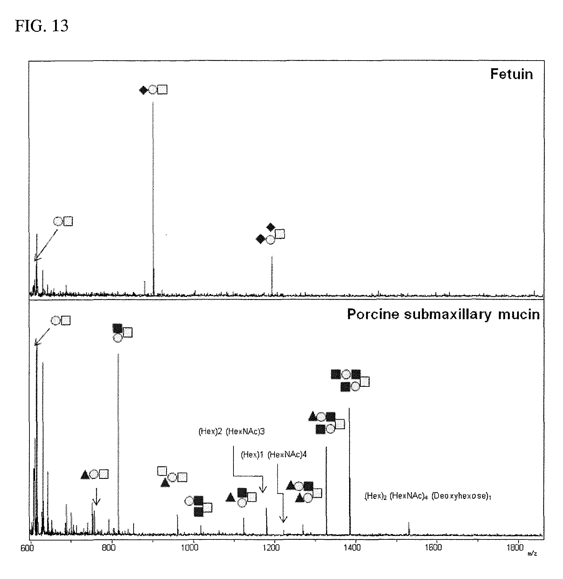

Fifty micrograms each of bovine fetuin or porcine submandibular gland mucin was weighed out, and 50 µL of water, 100 µL of a 0.5 M DP solution, and 50 µL of 0.6 M NaOH were added; thereafter, the respective mixtures were allowed to stand at 70°C for 16 hours. After completion of the reaction, the respective reaction mixtures were neutralized, and a quarter of each reaction mixture (50 µL) was subjected to purification of its sugar chain moiety using Dowex 50X8 and ZipTipC18. After evaporated to dryness, the resulting products were each dissolved in DHB and then analyzed by MALDI-TOF(/TOF) (UltraFlex II, Bruker). The results are shown in FIG. 13.

FIG. 13 shows the results obtained when bovine fetuin and porcine submandibular gland mucin, which were known to have O-linkedsugar chains, were profiled for their O-linked sugar chains using the technique of the present invention. Any of the results were qualitatively and quantitatively comparable to those previously reported in publications and the like, and this fact demonstrated that the present invention is useful for analysis of O-linked sugar chains in glycoproteins. As for the bovine fetuin, which was known to contain both O-linked sugar chains and N-linked sugar chains, only the O-linkedsugar chains were detected according to the inventive technique, with no N-linked sugar chains detected at all. These results demonstrated that the technique of this invention is not effective on N-linked sugar chains but has a highly selective effect on O-linkedsugar chains.

### [Synthesis Examples]

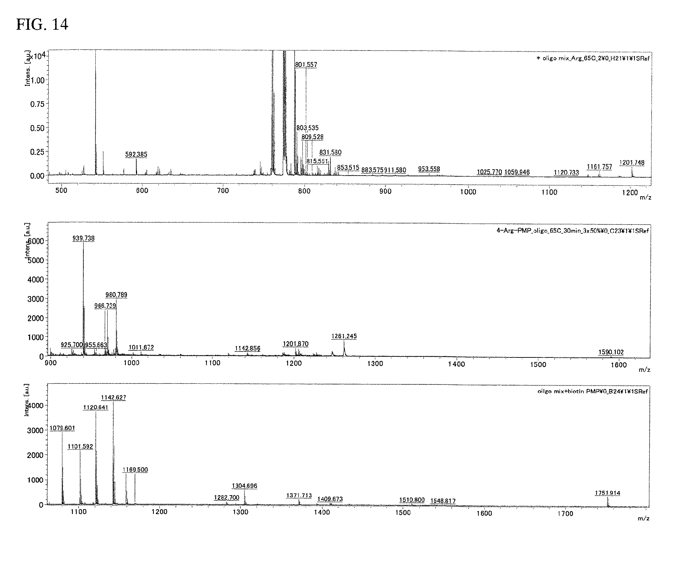

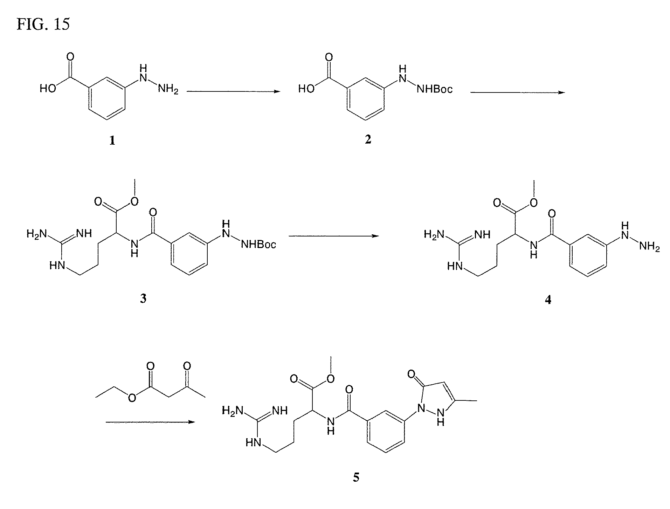

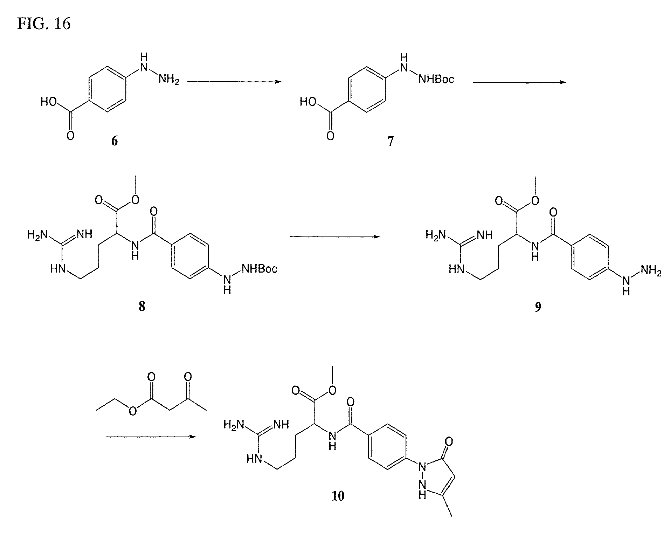

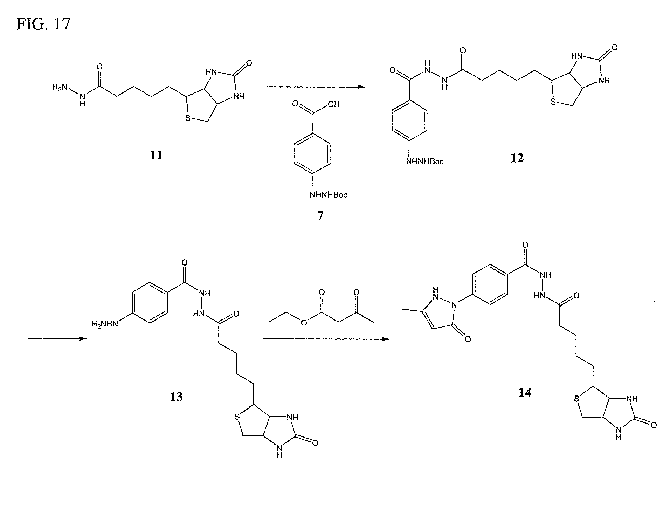

FIG. 14 shows the mass spectra of the sugar chains labeled with novel pyrazolone reagents. It was confirmed that in the presence of any of the reagents having a pyrazolone backbone, the sugar chains were mono- or bis-derivatized. FIGs. 15, 16 and 17 show the synthesis schemes of the novel pyrazolone reagents.

### (Synthesis Example 1)

To 10 µL of each mixture of 1 mM glucose, N-acetylglucosamine, N-acetyllactosamine, and chitotetraose, 20 µL of 0.45 M NaOH was first added and then 20 µL of any of 0.5 M synthetic pyrazolone reagents (Compound 5, 10 or 14) was added; thereafter, the respective mixtures were allowed to stand at 85°C for 2 hours. After completion of the reaction, the respective reaction mixtures were diluted and mixed with DHB, and the mixtures were analyzed by MALDI-TOF(/TOF) (UltraFlex II, Bruker).

### (Synthesis Example 2)

First, 3-hydrazinobenzoic acid (592 mg, 3.9 mmol) was dissolved in 10 mL of dimethylformamide, and di-t-butyl dicarbonate (854 mg, 3.9 mmol) was added; thereafter, the mixture was reacted at room temperature for 16 hours. The reaction mixture was subjected to extraction with ethyl acetate, and the extract was washed with 10 mM hydrochloric acid and water. The extract was concentrated to give the product of interest, Boc-3-hydrazinobenzoic acid (980 mg, 100%).

Boc-3-hydrazinobenzoic acid (491 mg, 1.95 mmol) was dissolved in 5 mL of methanol, and WSC (560 mg, 2.93 mmol) was added (Solution 1). Methyl arginine dihydrochloride (509 mg, 1.95 mmol) was dissolved in 2 mL of water, and triethylamine (395 mg, 3.9 mmol) was added. The resulting solution was mixed with Solution 1, and the mixture was reacted at room temperature for 16 hours. The reaction mixture was concentrated, and the concentrate was separated by silica gel column chromatography (chloroform:methanol, 9:1 →5:1) to give the product of interest, Boc-3-hydrazinobenzoyl arginine methyl ester (406 mg, 49%). In the analysis by MALDI-TOF-MS, the [M+H]⁺ ions of the product of interest were observed at m/z 423.4.

Four milliliters of trifluoroacetic acid was added to Boc-3-hydrazinobenzoyl arginine methyl ester (386 mg, 0.91 mmol), and the solution was reacted at room temperature for an hour; thereafter, the solvent was removed by concentration to quantitatively give the product of interest, 3-hydrazinobenzoyl arginine methyl ester. In the analysis by MALDI-TOF-MS, the [M+H]⁺ ions of the product of interest were observed at m/z 323.2.

Next, 3-hydrazinobenzoyl arginine methyl ester (29 mg, 0.09 mmol) was dissolved in 1 mL of ethanol, and 1 M ethyl acetoacetate in ethanol (80 µL, 0.08 mmol) as well as 200 µL of acetic acid were added; thereafter, the mixture was reacted at 65°C for 30 minutes. The reaction mixture was concentrated, and the concentrate was separated by silica gel column chromatography (chloroform:methanol, 9:1 → 5:1) to give the final product of interest, 3-arginine-added pyrazolone (21.7 mg, 70%). In the analysis by MALDI-TOF-MS, the [M+H]⁺ ions of the product of interest were observed at m/z 389.2.

### (Synthesis Example 3)

First, 4-hydrazinobenzoic acid (1.52g, 10 mmol) was dissolved in 10 mL of dimethylformamide, and di-t-butyl dicarbonate (2.18g, 10 mmol) was added; thereafter, the mixture was reacted at 65°C for an hour. The reaction mixture was subjected to extraction with ethyl acetate, and the extract was washed with 10 mM hydrochloric acid and water. The extract was concentrated to give the product of interest, Boc-4-hydrazinobenzoic acid.

Boc-4-hydrazinobenzoic acid (252 mg, 1.00 mmol) was dissolved in 10 mL of methanol, and 4-(4,6-dimethoxy-1,3,5-triazin-2-yl)-4-methylmorpholinium chloride (295 mg, 1.00 mmol) was added (Solution 1). Methyl arginine dihydrochloride (261 mg, 1.00 mmol) was dissolved in 2 mL of water, and triethylamine (100 µL, 1.00 mmol) was added. The resulting solution was mixed with Solution 1, and the mixture was reacted at room temperature for 2 hours. The reaction mixture was concentrated, and the concentrate was separated by silica gel column chromatography (chloroform:methanol, 9:1 →5:1) to give the product of interest, Boc-4-hydrazinobenzoyl arginine methyl ester (417 mg, 99%). In the analysis by MALDI-TOF-MS, the [M+H]⁺ ions of the product of interest were observed at m/z 423.3.

Four milliliters of trifluoroacetic acid was added to Boc-4-hydrazinobenzoyl arginine methyl ester (417 mg, 0.99 mmol), and the mixture was reacted at room temperature for 30 minutes; thereafter, the solvent was removed by concentration to quantitatively give the product of interest, 4-hydrazinobenzoyl arginine methyl ester. In the analysis by MALDI-TOF-MS, the [M+H]⁺ ions of the product of interest were observed at m/z 323.2.

Next, 4-hydrazinobenzoyl arginine methyl ester (159 mg, 0.49 mmol) was dissolved in 5 mL of ethanol, and 1 M ethyl acetoacetate in ethanol (450 µL, 0.45 mmol) as well as 1000 µL of acetic acid were added; thereafter, the mixture was reacted at 65°C for 30 minutes. The reaction mixture was concentrated, and the concentrate was separated by silica gel column chromatography (chloroform:methanol, 9:1 → 5:1) to give the final product of interest, 4-arginine-added pyrazolone (175 mg, 92%). In the analysis by MALDI-TOF-MS, the [M+H]⁺ ions of the product of interest were observed at m/z 389.2.

### (Synthesis Example 4)

Boc-4-hydrazinobenzoic acid (117 mg, 0.46 mmol) was dissolved in 5 mL of methanol, and 4-(4,6-dimethoxy-1,3,5-triazin-2-yl)-4-methylmorpholinium chloride (137 mg, 0.47 mmol) was added. Further, biotin hydrazide (1) (100 mg, 0.39 mmol) dissolved in 5 mL of dimethyl sulfoxide was added, and then the mixture was reacted at room temperature for 16 hours. The reaction mixture was concentrated, and the concentrate was separated by silica gel column chromatography (chloroform:methanol, 9:1 → 5:1) to give the product of interest, N-(Boc-4-hydrazinobenzoyl)-biotin hydrazide (2) (120 mg, 63%). In the analysis by MALDI-TOF-MS, the [M+-Na]⁺ ions of the product of interest were observed at m/z 515.4.

Four milliliters of trifluoroacetic acid was added to N-(Boc-4-hydrazinobenzoyl)-biotin hydrazide (2) (120 mg, 0.24 mmol), and the mixture was reacted at room temperature for 30 minutes; thereafter, the solvent was removed by concentration to quantitatively give the product of interest, N-(4-hydrazinobenzoyl)-biotin hydrazide (3). In the analysis by MALDI-TOF-MS, the [M+Na]⁺ ions of the product of interest were observed at m/z 415.2.

N-(4-hydrazinobenzoyl)-biotin hydrazide (3) (39 mg, 0.1 mmol) was dissolved in 1 mL of ethanol, and 1 M ethyl acetoacetate in ethanol (90 µL, 0.09 mmol) as well as 200 µL of acetic acid were added; thereafter, the mixture was reacted at 65°C for 30 minutes. The reaction mixture was concentrated, and the concentrate was separated by silica gel column chromatography (chloroform:methanol, 9:1 → 5:1) to give the final product of interest, biotin-added pyrazolone (4) (38 mg, 92%). In the analysis by MALDI-TOF-MS, the

[M+Na]⁺ ions of the product of interest were observed at m/z 459.4.

### INDUSTRIAL APPLICABILITY

The present invention provides a process that is very effective for analyzing O linked sugar chains -- the development of a methodology for this analysis has been long awaited. This invention also gives a new option to the BEMA method which has been conventionally used for phosphorylation analysis. This invention, which enables comprehensive analysis of post-translational modifications of serine and threonine, is expected to make a significant contribution to various fields including biochemistry, biology, pharmacy, drug discovery, and medicine.

## Claims

1. A process for cleaving a post-translational modification group from a serine residue and/or a threonine residue in a post-translationally modified glycoprotein and/or glycopeptide, wherein the glycoprotein and/or the glycopeptide which are a test substance as well as a labeling agent are dissolved or dispersed in a solvent and heat is applied under a basic condition, wherein the post-translational modification group from a serine residue and/or a threonine residue in the test substance is selectively cleaved and labeled, wherein the labeling agent comprises, as active ingredient, at least a pyrazolone derivative and wherein the post-translational modification group is an O-glycan.

2. The process according to Claim 1, wherein the post-translational modification group is labeled simultaneously with cleavage.

3. The process according to any one of Claims 1 and 2, wherein the serine residue and/or the threonine residue is/are labeled simultaneously upon cleavage.

4. The process according to any one of Claims 1-3, which is performed under a condition where the glycoprotein and/or the glycopeptide as well as the labeling agent are dissolved in water.

5. The process according to any one of Claims 1-4, wherein the labeling agent is used in combination with a thiol compound.

6. The process according to Claim 5, wherein the thiol compound is dithiothreitol.

7. A process for analyzing a post-translational modification, wherein after a post-translational modification group which is an O-glycan is cleaved from a serine residue and/or a threonine residue in a test substance and is labeled by the process according to any one of Claims 1-6, the post-translational modification group is subjected to liquid chromatography analysis and/or mass spectrometry analysis.

8. A process for analyzing a post-translational modification, wherein after a post-translational modification group which is an O-glycan is cleaved from a serine residue and/or a threonine residue in a test substance and the test substance is labeled by the process according to any one of Claims 1-6, the test substance is subjected to liquid chromatography analysis and/or mass spectrometry analysis.

9. A process for analyzing a post-translational modification, wherein the analyses as recited in Claims 7 and 8 are performed at the same time.

## Patentansprüche

1. Verfahren zum Abspalten einer post-translationalen Modifikationsgruppe von einem Serinrest und/oder einem Threoninrest in einem post-translational modifizierten Glycoprotein und/oder Glycopeptid, wobei das Glycoprotein und/oder das Glycopeptid, welche eine Testsubstanz sind, sowie ein Markierungsmittel in einem Lösungsmittel aufgelöst oder dispergiert werden und Hitze unter einer basischen Bedingung angewandt wird, wobei die post-translationale Modifikationsgruppe von einem Serinrest und/oder einem Threoninrest in der Testsubstanz selektiv abgespalten und markiert wird, wobei das Markierungsmittel als wirksamen Bestandteil mindestens ein Pyrazolonderivat umfasst, und wobei die post-translationale Modifikationsgruppe ein O-Glycan ist.

2. Verfahren gemäß Anspruch 1, wobei die post-translationale Modifikationsgruppe gleichzeitig mit der Abspaltung markiert wird.

3. Verfahren gemäß mindestens einem der Ansprüche 1 und 2, wobei der Serinrest und/oder der Threoninrest gleichzeitig nach Abspaltung markiert wird/werden.

4. Verfahren gemäß mindestens einem der Ansprüche 1 bis 3, welches unter einer Bedingung durchgeführt wird, wo das Glycoprotein und/oder das Glycopeptid sowie das Markierungsmittel in Wasser aufgelöst sind.

5. Verfahren gemäß mindestens einem der Ansprüche 1 bis 4, wobei das Markierungsmittel in Kombination mit einer Thiolverbindung verwendet wird.

6. Verfahren gemäß Anspruch 5, wobei die Thiolverbindung Dithiothreitol ist.

7. Verfahren zum Analysieren einer post-translationalen Modifikation, wobei, nachdem eine post-translationale Modifikationsgruppe, welche ein O-Glycan ist, von einem Serinrest und/oder einem Threoninrest in einer Testsubstanz abgespalten wird und durch das Verfahren gemäß mindestens einem der Ansprüche 1 bis 6 markiert wird, die post-translationale Modifikationsgruppe Flüssigchromatographieanalyse und/oder Massenspektrometrieanalyse unterzogen wird.

8. Verfahren zum Analysieren einer post-translationalen Modifikation, wobei, nachdem eine post-translationale Modifikationsgruppe, welche ein O-Glycan ist, von einem Serinrest und/oder einem Threoninrest in einer Testsubstanz abgespalten wird und die Testsubstanz durch das Verfahren gemäß mindestens einem der Ansprüche 1 bis 6 markiert wird, die Testsubstanz Flüssigchromatographieanalyse und/oder Massenspektrometrieanalyse unterzogen wird.

9. Verfahren zum Analysieren einer post-translationalen Modifikation, wobei die in den Ansprüchen 7 und 8 genannten Analysen zur gleichen Zeit durchgeführt werden.

## Revendications

1. Procédé de clivage d'un groupe de modifications post-traductionnelles à partir d'un résidu sérine et/ou d'un résidu thréonine dans une glycoprotéine et/ou un glycopeptide modifiés après traduction, dans lequel la glycoprotéine et/ou le glycopeptide qui sont une substance de test ainsi qu'un agent de marquage sont dissous ou dispersés dans un solvant et de la chaleur est appliquée dans des conditions basiques, dans lequel le groupe de modifications post-traductionnelles à partir d'un résidu sérine et/ou d'un résidu thréonine dans la substance de test est sélectivement clivé et marqué, dans lequel l'agent de marquage comprend, en tant qu'ingrédient actif, au moins un dérivé de pyrazolone et dans lequel le groupe de modifications post-traductionnelles est un O-glycane.

2. Procédé selon la revendication 1, dans lequel le groupe de modifications post-traductionnelles est marqué simultanément avec le clivage.

3. Procédé selon l'une quelconque des revendications 1 et 2, dans lequel le résidu sérine et/ou le résidu thréonine est/sont marqués simultanément lors du clivage.

4. Procédé selon l'une quelconque des revendications 1 à 3, qui est réalisé dans des conditions où la glycoprotéine et/ou le glycopeptide ainsi que l'agent de marquage sont dissous dans de l'eau.

5. Procédé selon l'une quelconque des revendications 1 à 4, dans lequel l'agent de marquage est utilisé en combinaison avec un composé de thiol.

6. Procédé selon la revendication 5, dans lequel le composé de thiol est le dithiothréitol.

7. Procédé d'analyse d'une modification post-traductionnelle, dans lequel après qu'un groupe de modifications post-traductionnelles qui est un O-glycane est clivé à partir d'un résidu sérine et/ou d'un résidu thréonine dans une substance de test et est marqué par le procédé selon l'une quelconque des revendications 1 à 6, le groupe de modifications post-traductionnelles est soumis à une analyse par chromatographie liquide et/ou à une analyse par spectrométrie de masse.

8. Procédé d'analyse d'une modification post-traductionnelle, dans lequel après qu'un groupe de modifications post-traductionnelles qui est un O-glycane est clivé à partir d'un résidu sérine et/ou d'un résidu thréonine dans une substance de test et que la substance de test est marquée par le procédé selon l'une quelconque des revendications 1 à 6, la substance de test est soumise à une analyse par chromatographie liquide et/ou à une analyse par spectrométrie de masse.

9. Procédé d'analyse d'une modification post-traductionnelle, dans lequel les analyses selon les revendications 7 et 8 sont réalisées en même temps.
